# EUROPEAN PATENT APPLICATION

(11) **EP 3 744 286 A1**
(43) Date of publication of application: **02.12.2020**
(21) Application number: 19176728.4
(22) Date of filing: 27.05.2019
(51) Int. Cl.: A61B 90/20

(54) **MICROSCOPE SYSTEM AND METHOD FOR CONTROLLING A SURGICAL MICROSCOPE**

(71) Applicant: Leica Instruments (Singapore) Pte. Ltd., 608924 Singapore (SG)
(72) Inventor: Themelis, George, 88131 Lindau (DE)
(74) Representative: Schaumburg und Partner Patentanwälte mbB

(57) **Abstract**

A microscope system comprises a surgical microscope, an operating device configured to be operated by a user, and a processor configured to receive a command signal from the operating device in response to the user operation and to control the surgical microscope based on the command signal, wherein the operating device comprises a sensor unit configured to detect the user operation and to generate the command signal based on the detected user operation, wherein the sensor unit is further configured to be worn by the user.

## Description

### Technical field

The present invention relates to a microscope system and a method for controlling a surgical microscope.

### Background

Surgical microscopes have been widely used to carry out surgical procedures on a patient. When using such a surgical microscope, every operation of the microscope, e.g. repositioning, focusing, zooming, etc., requires a physical interaction of the surgeon with the microscope. For instance, the surgeon has to press buttons and to grab handles in order to cause the surgical microscope to perform the intended operation. Manually operating the surgical microscope is particularly cumbersome as the surgeon typically needs to interrupt his work and free his hands from the surgical instruments to operate the surgical microscope as intended. After having adjusted the surgical microscope, the surgeon must pick up the surgical tools and find again the target spot he was working on before manually operating the microscope. Such an interruption results in a combination of delays and suboptimal microscope settings as the surgeon e.g. prefers to continue the work with a suboptimal setting, for example poor focus, rather than to interrupt the work for optimizing the setting. Further, whereas it might be possible to use another specialist as an assistant who manually operates the microscope, such an approach has its disadvantages in terms of costs and limited space in an operating room.

### Summary

Recently, a microscope system using a gesture control has been proposed in document EP 3 285 107 A1. However, the present inventors have found that such a gesture control has its limit as the gestures are detected from a distance of the user. Accordingly, the inventors found that gesture recognition might not be possible at certain detection angles and/or positions. For example, it is not suitable to locate a camera below the surgical table in order to detect the position and movements of the user's legs or feet. Further, the inventors found that gesture recognition might be inaccurate in some situations such as when the user's body is covered by clothes and thereby fine movements might not be visible. Further, the inventors found that in general optical recognition of user gestures are prone to errors caused by accidental movements of the surgeon or movements by other persons in the operating room.

Therefore, it is an object of the present invention to provide a microscope system and a method for controlling a surgical microscope enabling the user to operate a surgical microscope efficiently without or at least only minimally interrupting the surgical procedure.

The afore-mentioned object is achieved by the subject-matter of the independent claims. Advantageous embodiments are defined in the dependent claims.

The proposed microscope system comprises a surgical microscope, an operating device configured to be operated by a user, and a processor configured to receive a command signal from the operating device in response to the user operation and to control the surgical microscope based on the command signal. The operating device comprises a sensor unit configured to detect the user operation and to generate the command signal based on the detected user operation. The sensor unit is further configured to be worn by the user.

As the microscope system comprises a sensor unit which is configured to be worn by the user (e.g. a surgeon or nurse operating the microscope), the microscope system allows a detection of the user operation on the spot, i.e. at the location where the user operates the operating device in order to control the microscope system. Thus, disadvantages associated with detecting the user operation from a distance are avoided. In particular, the proposed microscope system enables recognition of the user operation irrespective of the user's position and irrespective of whether or not the user or any other person in the operating room is moving. Accordingly, the user is enabled to control the microscope system easier, faster and more precise and reliable than before.

The configuration of the sensor unit included in the operating device is not limited to specific embodiments provided that the configuration enables the sensor unit to be worn by the user. For this, the sensor unit may be formed such that it can be attached to a part of the user's body, e.g. a finger, a hand, an arm, the head, a leg, and/or a foot. For instance, the sensor unit may be formed as a finger ring, a bracelet, a wrist band, a head band, a foot strap, a knee strap etc. In particular, the microscope system may comprise a user wearable device configured to be worn on a part of the body of the user, said user wearable device including the sensor unit. In this respect, the wearable device may be configured to exclusively serve to detect the user operation performed on the operating device. Alternatively, the user wearable device may also be configured to provide additional functions other than the intended sensor function so that the sensor unit only forms one of a plurality of different functional elements of the user wearable device.

Preferably, the sensor unit comprises at least one sensor selected from a group comprising a gyroscope, an accelerometer, a muscle activity sensor, a button, a pressure sensor, a magnetic field sensor, an electric field sensor, an induction sensor, and a microphone. A gyroscope and an accelerometer may e.g. be used to detect a turning movement of an arm of the user in case that the operating device including the afore-mentioned component is part of a bracelet worn on the user's arm. By detecting the arm movement, it is e.g. possible to adjust the focus of an optical system of a surgical microscope as intended. Needless to say that any other kind of microscope operation can be controlled in such a way.

In a preferred embodiment, the sensor unit comprises at least two sensors from the group, said two sensors being configured to interact with each other. For example, a pressure sensor may be combined with a muscle activity sensor in order to increase the accuracy with which the user operation is detected.

In a further preferred embodiment, one of the two sensors is configured to detect an activation of the sensor unit, and the other sensor is configured to generate the command signal based on the user input after detecting the activation of the sensor unit. By using two sensors as mentioned above, accidentally invoking a command signal may be avoided.

Preferably, the sensor unit may be configured to be a stand-alone sensor unit, i.e. a unit which does need a counterpart device. Such a stand-alone sensor unit may e.g. be formed by an accelerometer. Alternatively, the sensor unit may be configured to be a part of a sensor system including a counterpart device. For instance, the sensor unit may be an electric field sensor which is to be worn by the user and which is configured to measure changes of an electric field created by the counterpart device. Preferably, the microscope system comprises a further, i.e. additional sensor unit configured to interact with said sensor unit to generate the command signal. This additional sensor unit may be used to improve the accuracy for detecting the user operation. Further, the sensor unit to be worn by the user may be combined with said further sensor unit to perform a complete task. For instance, the user may use a voice command to activate the use of a gyroscope or accelerometer for adjusting the focus of the surgical microscope. According to another example, the activation of a gyroscope may be done by pressing a button wherein this button is carried by a shoe worn on one foot of the user and being pressed by the other foot.

In a preferred embodiment, the sensor unit comprises a feedback module configured to provide a feedback information on an operating state. Such a feedback module may be used to confirm that the sensor unit to be worn by the user is now being used to transmit the command signal to the processor.

Preferably, the feedback information comprises at least one of an activation state of the sensor unit and an operating state of the surgical microscope. For instance, the feedback module may confirm that a mode for generating the command signal ends, and the sensor unit is now inactive. As a further example, the feedback module may provide the user with information indicating that an attempt to initiate the sensor unit was not successful. Further, any message from the surgical microscope may be converted by the feedback module in a corresponding feedback information on an operating state which is to be brought to the user's attention. Such an operating state may be for example a state in which a zoom cannot be increased further or an overheat of a light source occurs.

The feedback module may be configured to provide said feedback in form of at least one of vibration, mechanical pressure, electrical pulse, sound and light.

Preferably, the sensor unit further comprises a control element configured to control the surgical microscope in a manner not related to the generation of said command signal, i.e. in a manner going beyond an operation of the surgical microscope which is caused by the user during surgery e.g. for zooming, focusing, repositioning, etc.

Preferably, the control element is configured to detect a user identification. For example, the control element for detecting the user identification may comprise an RFID (contactless) chip. In such a case, the RFID chip used for identifying the user may also contain the sensor unit which detects the user operation and generates the command signal based on which the surgical microscope is controlled as intended by the user. The sensor unit may e.g. be configured to detect electric or magnetic fields in order to recognize gestures. By combining the RFID chip and the sensor unit, it is possible to increase functionality of the microscope system. In particular, the microscope system may be able to distinguish the input from multiple users and even allow collaborative actions between multiple users.

In a preferred embodiment, the sensor may be configured to detect a physical condition of the user. For instance, the sensor unit may be formed to measure fatigue, stress, tremor, stability, and thereby assess a health condition of the surgeon, the ability to continue the surgery, etc.

According to another aspect, a method for controlling a surgical microscope is provided, comprising the following steps: detecting a user operation by means of a sensor unit which is worn by a user, generating a command signal based on the detected user operation, and controlling the surgical microscope based on the command signal.

According to a further aspect, a computer program is provided with a program code for performing the method, when the computer program is run on a processor.

### Short Description of the Figures

Hereinafter, preferred embodiments are described with reference to the drawings, in which:
Figure 1 is a diagram illustrating a configuration of a microscope system according to an embodiment; and
Figure 2 is a flow diagram illustrating an exemplary method for controlling a surgical microscope included in the microscope system of Figure 1.

### Detailed Description

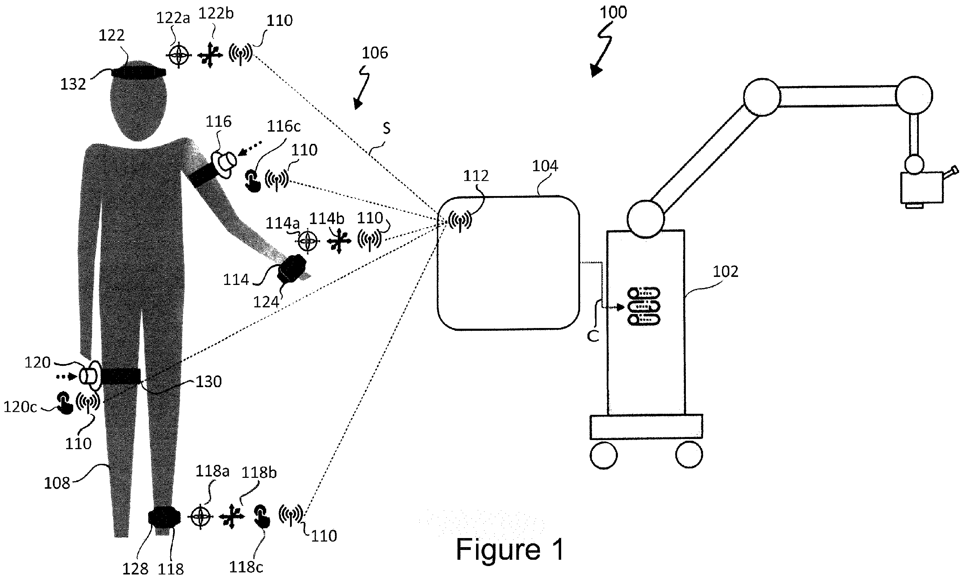

Figure 1 is a diagram showing a microscope system 100 comprising a surgical microscope 102 and a processor 104. The processor 104 may be included in a control device such as a computer which is physically separated from the surgical microscope 102. Alternatively, the processor 104 may be integrated with the surgical microscope 102.

The microscope system 100 further comprises an operating device 106 which is operated by a user 108 when performing surgery on a patient. For instance, the user 108 may operate the operating device 106 to control the surgical microscope 102 in terms of repositioning, focusing, zooming, etc. For this, the operating device 106 is configured to generate a command signal S in response to the user operation and to transmit the command signal S to the processor 104. Preferably, the transmission of the command signal S from the operating device 106 to the processor 104 is conducted by means of a wireless communication. Accordingly, the operating device 106 and the processor 104 may include corresponding wireless communication means 110 and 112, respectively.

The operating device 106 comprises at least one sensor unit which is configured to detect the user operation and to generate the command signal S based on the detected user operation. For the purpose of illustration, Figure 1 shows a plurality of sensor units 114, 116, 118, 120, 122 which might be used alone or in combination for detecting the user operation and generating the command signal. In particular, it is sufficient to use only one of the sensor units 114 to 122. Nevertheless, circumstances are conceivable in which using more than one single sensor unit may have some benefits, in particular when the control of the surgical microscope 102 to be executed by the user 108 becomes relatively complex.

According to the example shown in Figure 1, different types of sensors may be provided, for instance a gyroscope, an accelerometer and a button. Each of the sensor units 114 to 122 may comprise one or more of these sensor types. In Figure 1, the different sensor types are referred to by different suffices "a", "b" and "c", wherein "a" designates a gyroscope sensor type, "b" an accelerometer sensor type, and "c" a button sensor type. Accordingly, reference signs 114a and 114b shall e.g. illustrate that the sensor unit 114 may be formed by a gyroscope and an accelerometer, respectively. The same applies to the other sensor units 116 to 122.

Each of the sensor units 114 to 122 is configured to be worn by the user 108. For instance, the sensor unit 114 is included in a user wearable device 124 which is configured to be worn on a wrist of the user 108. Thus, the user wearable device 124 may be formed by a wrist band.

Correspondingly, the sensor unit 116 is included in a user wearable device 126 to be worn on an upper arm of the user 108. Thus, the user wearable device 126 may be formed by a bracelet. The sensor unit 118 is included in a user wearable device 128 which is to be worn on an ankle of the user 108. Accordingly, the user wearable device 128 may be formed by a foot strap. The sensor device 120 is included in a user wearable device 130 to be worn on a knee of the user 108. Accordingly, the user wearable device 130 may be formed by a knee strap. Finally, the sensor unit 122 is included in a user wearable device 132 to be worn on the head of the user 108. Thus, the user wearable device 132 may be formed by a headband.

As can be seen in Figure 1, the sensor unit 114 may comprise a gyroscope 114a and/or an accelerometer 114b. Likewise, the sensor unit 116 may comprise a button 116c, the sensor unit 118 may comprise a gyroscope 118a, an accelerometer 118b and/or a button 118c. The sensor unit 120 may comprise a button 120c. The sensor unit 122 may comprise a gyroscope 122a and/or an accelerometer 122b.

The sensors shown in Figure 1 are to be understood merely as examples. Other sensor types may be used, e.g. muscle activity sensors, pressure sensors, magnetic fields sensors, electric field sensors, induction sensors, and microphones.

As illustrated in the embodiment shown in Figure 1, different sensor types may be combined in order to increase the accuracy with which the user operation is detected. For instance, the accuracy of detecting a hand movement may be increased in case that the sensor unit 114 uses both the gyroscope 114a and the accelerometer 114b. Likewise, one of the afore-mentioned sensors may be configured to detect an activation of the sensor unit 114, whereas the other sensor may generate the command signal S after the activation of the sensor unit 114 has been detected.

The sensor units 114 to 122 shown in Figure 1 are configured to be stand-alone sensor units. However, each of the sensor units 114 to 122 may be configured to interact with a corresponding counterpart device.

Further, each of the sensor units 114 to 122 may comprise a feedback module providing a feedback information based on which the user 108 is enabled to detect a specific operating state of the surgical microscope 102. For instance, such a feedback module may inform the user 108 that an attempt to initiate the corresponding sensor unit failed.

Further, the sensor units 114 to 122 may be configured to measure a physical condition of the user 108, e.g. fatigue, stress, tremor, and stability.

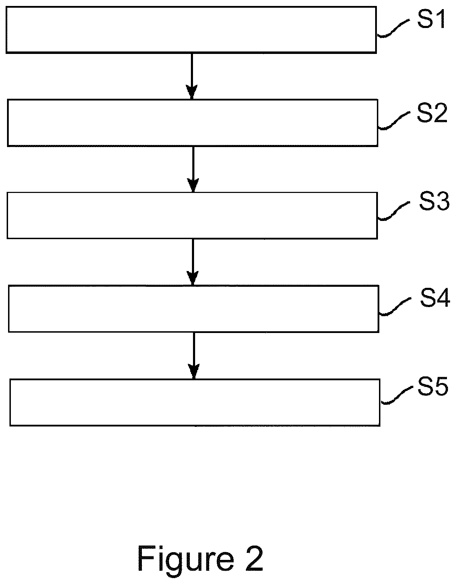

Figure 2 shows a flow diagram illustrating an example of a method for controlling the surgical microscope 102. For the example shown in Figure 2, it is assumed that a hand movement of the user 108 is detected by the gyroscope 114a included in the wrist band 124.

In step S1, the user 108 moves his hand in order to control the surgical microscope 102 in a specific way, e.g. for focusing onto a target area of a patient to be examined.

In step S2, the gyroscope 114a detects the hand movement performed by the user 108 and generates the command signal S based on the detected hand movement.

In step S3, the gyroscope 114a transmits the command signal S to the processor 104 via wireless communication using the communication means 110 and 112.

In step S4, the processor generates a control signal C and transmits the control signal C to the surgical microscope.

In step S5, the surgical microscope 102 performs the operation intended by the user based on the control signal C. For example, the surgical microscope 102 actuates a drive mechanism for focusing an optical system onto the target area.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus. Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of Reference Signs

- 100: microscope system
- 102: surgical microscope
- 104: processor
- 106: operating device
- 108: user
- 110, 112: wireless communication means
- 114: sensor means
- 114a: gyroscope
- 114b: accelerometer
- 116: sensor means
- 116c: button
- 118: sensor means
- 118a: gyroscope
- 118b: accelerometer
- 118c: button
- 120: sensor unit
- 120c: button
- 122: sensor means
- 122a: gyroscope
- 122b: accelerometer
- 124: wrist band
- 126: bracelet
- 128: foot strap
- 130: knee strap
- 132: headband
- S: command signal
- C: control signal

## Claims

1. A microscope system (100), comprising:
a surgical microscope (102),
an operating device (106) configured to be operated by a user (108), and
a processor (104) configured to receive a command signal (S) from the operating device (106) in response to the user operation and to control the surgical microscope (102) based on the command signal,
wherein the operating device (106) comprises a sensor unit (114 to 122) configured to detect the user operation and to generate the command signal (S) based on the detected user operation,
wherein the sensor unit (114 to 122) is further configured to be worn by the user (108).

2. The microscope system (100) according to claim 1, wherein the sensor unit (114 to 122) comprises at least one sensor selected from a group comprising a gyroscope, an accelerometer, a muscle activity sensor, a button, a pressure sensor, a magnetic field sensor, an electric field sensor, an induction sensor, and a microphone.

3. The microscope system (100) according to claim 2, wherein the sensor unit (114 to 122) comprises at least two sensors from the group, said two sensors being configured to interact with each other.

4. The microscope system (100) according to claim 3, wherein one of the two sensors is configured to detect an activation of the sensor unit (114 to 122), and the other sensor is configured to generate the command signal based on the user input after detecting the activation of the sensor unit (114 to 122).

5. The microscope system (100) according to one of the preceding claims, wherein the sensor unit (114 to 122) is configured to be a stand-alone sensor unit.

6. The microscope system (100) according to one of the claims 1 to 4, comprising a further sensor unit configured to interact with said sensor unit (114 to 122) to generate the command signal.

7. The microscope system (100) according to one of the preceding claims, wherein the sensor unit (114 to 122) comprises a feedback module configured to provide a feedback information on an operating state.

8. The microscope system (100) according to claim 7, wherein said feedback information comprises at least one of an activation state of the sensor unit (114 to 122) and an operating status of the surgical microscope (102).

9. The microscope system (100) according to claim 7 or 8, wherein the feedback module is configured to provide said feedback in form of at least one of vibration, mechanical pressure, electrical pulse, sound and light.

10. The microscope system (100) according to one of the preceding claims, wherein the sensor unit (114 to 122) further comprises a control element configured to control the surgical microscope in a manner not related to the generation of said command signal.

11. The microscope system (100) according to claim 10, wherein the control element is configured to detect a user identification.

12. The microscope system according to one of the preceding claims, wherein the sensor unit (114 to 122) is further configured to detect a physical condition of the user.

13. The microscope (100) system according to one of the preceding claims, comprising a user wearable device (124, 126, 128, 130, 132) configured to be worn on a part of the body of the user (108), said user wearable device including the sensor unit (114 to 122).

14. A method for controlling a surgical microscope (102), comprising the following steps:
detecting a user operation by means of a sensor unit (108) which is worn by a user (108),
generating a command signal (S) based on the detected user operation, and
controlling the surgical microscope (102) based on the command signal (S).

15. Computer program with a program code for performing the method according to claim 14, when the computer program is run on a processor (104).
